# EUROPEAN PATENT SPECIFICATION

(11) **EP 1 987 858 B1**
(45) Date of publication and mention of the grant of the patent: **03.08.2016**
(21) Application number: 08075255.3
(22) Date of filing: 31.03.2008
(51) Int. Cl.: A61N 1/372, A61N 1/37, A61B 5/00, G05B 19/042, G06F 19/00

(54) **Follow-up support system for implantable medical devices**
Nachbehandlungsstützsystem für implantierbare medizinische Vorrichtungen
Système de support de suivi pour dispositifs médicaux implantables

(30) Priority: 30.04.2007 US 742349
(43) Date of publication of application: 05.11.2008
(73) Proprietor: Biotronik CRM Patent AG, 6341 Baar (CH)
(72) Inventor: Kukla, Volker, 12157 Berlin (DE); Reinke, Joachim, 10439 Berlin (DE)
(74) Representative: Galander, Marcus

(56) References cited:
- EP-A1- 0 791 373
- EP-A1- 1 057 448
- EP-A2- 1 855 221
- US-A- 5 476 483
- US-A1- 2003 028 082
- US-A1- 2004 122 297
- US-A1- 2005 204 134
- US-A1- 2006 010 090

## Description

The invention refers to an integrated bi-directional data communication and follow-up system for at least one implanted medical device (IMD). The invention relates in particular to follow up systems for an implantable cardiac pacemaker an implantable pulse generator (IPG) or an implantable cardioverter/defibrillator (ICD).

Implantable heart stimulators can be used for treating a variety of heart disorders like bradycardia, tachycardia or fibrillation by way of electric stimulation pulses delivered to the heart tissue, the myocardium. Strong enough a stimulation pulse outside a heart chamber's refractory period leads excitation of the myocardium of that heart chamber, which in turn is followed by a contraction of the respective heart chamber.

Depending on the disorder to be treated, such heart stimulator generates electrical stimulation pulses that are delivered to the heart tissue (myocardium) of a respective heart chamber according to an adequate timing regime. Delivery of stimulation pulses to the myocardium is usually achieved by means of an electrode lead that is electrically connected to a stimulation pulse generator inside a heart stimulator's housing and that carries a stimulation electrode in the region of its distal end. A stimulation pulse also is called a pace. Similarly, pacing a heart chamber means stimulating a heart chamber by delivery of a stimulation pulse.

Parameters defining a therapy to be delivered by a heart stimulator need to be adapted to a patient's need. After adapting all parameters there may occur nevertheless irregularities such as unsuccessful attempts of treating a tachycardia by means of an antitachycardia therapy (antitachycardia pacing; ATP). This may necessitate reprogramming the antitachycardia therapy during a next follow up session.

IMDs may feature a telemetry unit for wireless access to the implanted medical device. Usually IMDs such as pacemakers offer a plurality of operation modes and feature a number of adjustable parameters to best match the IMDs configuration to a patient's individual need. Further, state of the art pacemakers collect a number of operational and physiological data such as, inter alia, data representing an intracardiac electogram. Operational data collected and stored by the IMD include irregularity indicators indicating irregularities such as incidents of tachycardia etc. These indicators are at least temporarily stored in an IMDs memory.

During follow-up sessions, a patient having an IMD meets a physician that checks the patient's health state and the state of the IMD. By means of a programmer or a similar device for wireless data communication with the IMD and an IMD's telemetry unit connected to the IMD's memory the physician is able to read out the IMD's memory and to reprogram the IMD if necessary.

Irregularities felt by the patient are noted by the physician during follow-up and may entice the physician to adapt the operational parameters of the IMD.

Between the follow-up sessions the IMD may wirelessly communicate data to an external device in the patient' proximity. The external device can be linked to a remote service center for collection of data collected or generated by the IMD.

Such systems are, for example, disclosed in US 2003/0028082 and EP 1 057 448.

The inventors have noted a number of problems that render dealing with irregularities during follow-up sessions inefficient:
During follow-up examination of patients with a cardiac implant (ICD, IPG) the examining physician has the problem to address all irregularities of the patient which occurred after the previous follow-up examination. This is difficult because
   1. The physician conducting the follow-up is not always the same person thus doesn't always know the complete patient history.
   2. The physician has possibly a lot of patients to examine per day. Time and schedule is tight.
   3. There is not only a short list of possible irregularities (together with standard solutions) which can occur, so that the task to solve irregularities is not easy.
   4. The information about irregularities of the patient has to be accessible at exactly that place where the patient is during follow-up examination and not where the clinic is located in which he received the implant.

Possible solutions and their disadvantages are:
1. The examining physician can ask the patient about symptoms he felt during the last months (after the previous follow-up).
   Disadvantage: Symptoms do not necessarily stand for irregularities, symptoms can be forgotten. Furthermore, irregularities sometimes are not symptomatic.
2. The examining physician can consult a clinical information system in which all irregularities of patients are entered as soon as they become known to any the clinic.
   Disadvantage: someone has to feed the clinical information system with irregularities. The patient must log all symptoms.
3. For each patient a paper file is kept which travels together with the patient as long as the patient has the implant.
   Disadvantage: The file might get lost. The patient might forget the file which will sooner or later render it incomplete (and thus useless).

It is an object of the invention to provide an integrated bi-directional data communication and follow-up system for at least one implanted medical device (IMD) that facilitates a follow-up session conducted by a physician.

According to the present invention the object of the invention is achieved by an IMD follow-up support unit for supporting a follow-up session for an individual implantable medical device (IMD). The IMD follow-up support unit comprises:
- a data input for receiving IMD data, said IMD data including an IMD identification code identifying an individual IMD and an IMD type, and IMD irregularity messages including data identifying type and date of an irregularity,
- a database for storing IMD data received via said data input and a follow-up history dataset comprising at least a date of a latest follow-up session for an individual IMD, an
a data processor having access to said data base.

The processor is adapted to:
- generate a list of irregularities which took place after a previous follow-up session,
- calculate an individual IMD irregularity index for each type of irregularity based on IMD irregularity messages associated to an individual IMD by way of said IMD identification code,
- calculate for each type of irregularity an average irregularity index over the same irregularity over all IMD irregularity messages associated to the same type of IMD,
- compare said calculated individual IMD irregularity index with said calculated average irregularity index and
- generate an indication if said individual IMD irregularity index significantly differs from said average irregularity index.

Thus, the follow up support unit is able to automatically generate useful information about irregularities that are not available to any device that only is considering information received from an individual IMD.

The irregularity index can be a composed variable or a one or more digit number.

Also it is possible to create and compare irregularity indices associated not only to the same type of irregularity but to the same IMD in order to compare alternative therapy mode or setups of an individual IMD. In such case individual irregularity indices would be create for different therapies or IMD setups in order to compare these setups with each other. Thus it is possible e.g. to determine which mode of antitachycardia therapy (ATP) is more successful for an individual patient, burst or ramp?

Preferably, the data processor is further adapted to calculate a list of hints upon generating said indication, wherein said list of hints is calculated on the basis of said irregularity messages stored in said data base and wherein said list of hints comprises at least one of:
- static suggestions which are always associated with the irregularity leading to said indication and
- dynamic suggestions, wherein said data processor is adapted to calculate for each dynamic suggestion an IMD setting index of the device settings currently configured to treat said irregularity and to compare said IMD setting index to an average index over all device settings of IMDs of this type.

Such suggestions can help the physician during a follow up session.

In a further preferred embodiment the IMD follow-up support unit is part of or is at least remotely connected to an IMD follow-up workstation comprising
- a display for displaying said hints calculated by said data processor
- input means for entering of commands and/or data by a user, and
- a telemetry link adapted for wireless transmission of data to an IMD.

The telemetry link may either be a direct link as is provided by a programmer directly communicating with an IMD or an indirect link via a remote service center.

The IMD follow-up support unit is adapted to generate display messages that include a hint generated and may include response buttons that allow a user to respond to said hints. The follow-up system can further be adapted to process a response and to eventually initiate reprogramming of the IMD according to the hint, if possible.

The response buttons can be actuated by means of e.g. a computer mouse. Alternatively, the display may be touch sensitive allowing direct actuation of the buttons displayed. Another alternative would be means for voice control of the IMD follow-up unit to respond to the messages displayed.

Preferably, the follow-up support system is adapted to gives the physician conducting the follow-up the possibility
- to rate the given hint as unimportant, preferably either for one time only or generally.
- to tell the assistant that the issue is being handled
- to automatically re-program the IMD as suggested.

It is further preferred that the follow-up support unit is adapted to group and priorize the irregularities according to an internally stored irregularity priority list and to initiate displaying of the irregularities highest priority first.

A further aspect of the invention relates to a work station (e.g., the programmer itself) comprising a follow up support unit as disclosed herein before. Alternatively, the follow-up support unit can be part of a remote service center that can be linked to an external device for communicating with an IMD. The external device preferably is a workstation that is adapted for wireless data communication with the IMD.

According to another aspect of the invention, the problem if solved by a method of generating support hints assisting in a follow-up session for an implantable medical device, said method comprising:
- selecting an IMD and indicating that a follow-up is about to be performed,
- generating a list of irregularities which took place after a previous follow-up, said list of irregularities containing every type of irregularity which was detected
- calculating for each detected irregularity an irregularity index which makes the irregularity inter-IMD comparable
- calculating for each detected irregularity an average index over the same irregularity over all patients which have the same type of IMD.
- comparing said calculated irregularity index with said calculated average index and displaying it to a user conducting the follow-up if said irregularity indices significantly differ from each other
- and, if there is a significant difference, calculating a list of hints which can help a user to improve therapy of this irregularity.

Preferably said hints comprise
- static suggestions which are always associated with the irregularity
- dynamic suggestions that are generated by calculating an index of the device setting currently configured to treat the irregularity and comparing it to an average index over all device settings of IMDs of this type.

The method preferably includes:
- giving the physician conducting the follow-up the possibility
   - to rate the given hint as unimportant (either for this time only or generally)
   - to tell the assistant that the issue is being handled
   - to automatically re-program the IMD as suggested.

Further, the method may include grouping and priorizing the irregularities according to a stored irregularity priority list and presenting the irregularities highest priority first.

It is to be appreciated that features of preferred embodiments of the invention may be combined in any useful manner thus arriving at further preferred embodiments of the invention not explicitly mentioned in this disclosure.

The above and other aspects, features and advantages of the present invention will be more apparent from the following more particular description thereof, presented in conjunction with the following drawings wherein:
- FIG. 1: is a system including an implantable medical device, and external device/programmer and a remote service center.
- FIG. 2: shows a dual chamber pacemaker/ atrial defibrillator/cardioverter connected to leads placed in a heart.

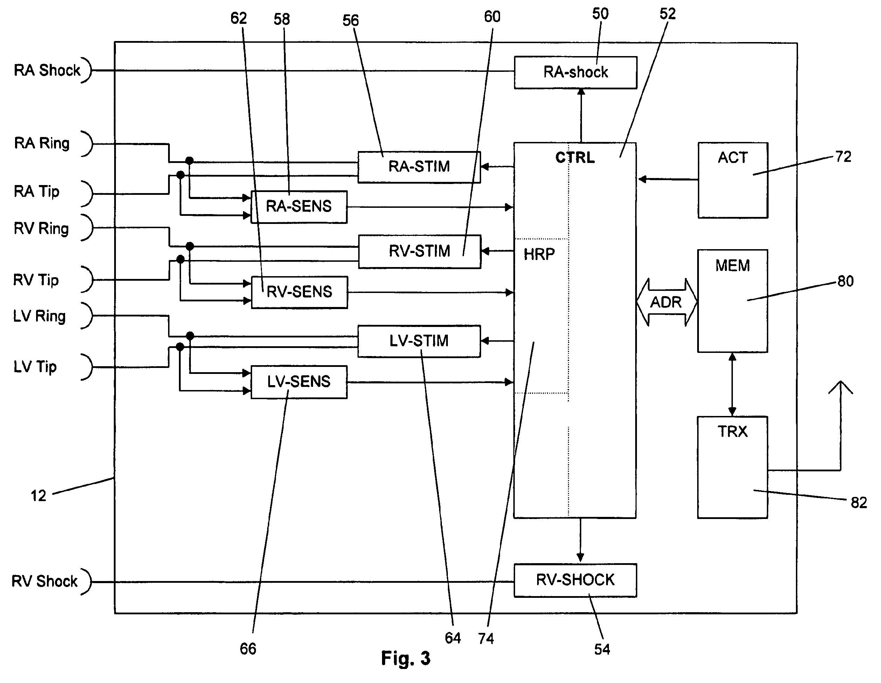

- FIG. 3: is a block diagram of the device of figure 1.
- FIG. 4: is a more detailed representation of an external device/programmer of the system of figure 1.

The following description is of the best mode presently contemplated for carrying out the invention. This description is not to be taken in a limiting sense, but is made merely for the purpose of describing the general principles of the invention. The scope of the invention should be determined with reference to the claims.

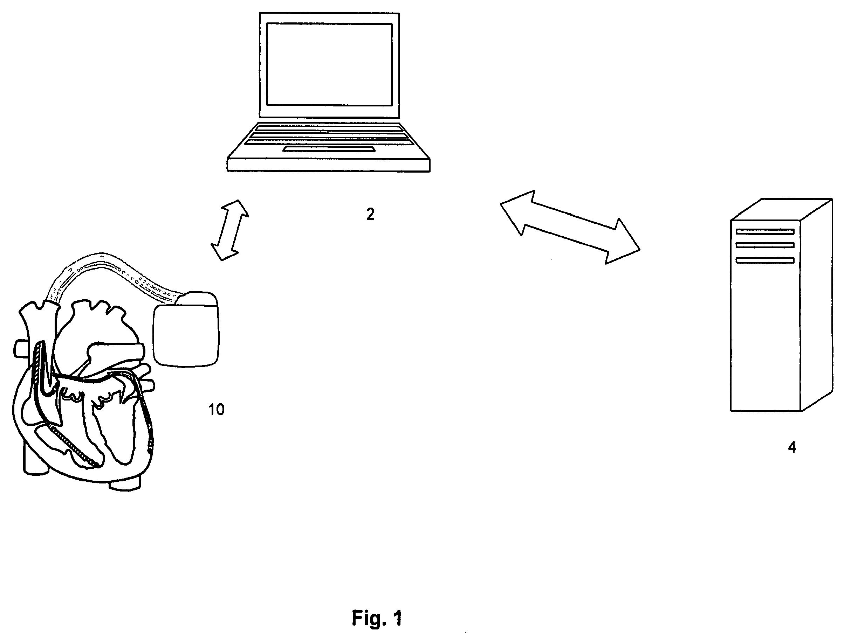

In figure 1, a system comprising an implantable medical device 10, an external device 2 and a remote service center 4 are shown. The implantable medical device 10 is an implantable heart stimulator that can act as a biventricular pacemaker and cardioverter/defibrillator. The heart stimulator 10 comprises a telemetry unit 82 for wireless data communication with the external device. The external device can wirelessly communicate with the heart stimulator 10 and thus can act as a programmer for the heart stimulator. Further, the external device 2 provides for a link to the remote service center 4 to upload data to the service center 4 and to download data from the service center 4. Thus it is possible that the external device 2 reads out data from the heart stimulator 10 and uploads the data to the service center. The external device 2 is a work station.

The service center collects data received from different external devices belonging to different heart stimulators. The service center processes these data in order to calculate irregularity indices. The data uploaded to the service center IMD includes an identification code identifying an individual IMD and an IMD type, and IMD irregularity messages including data identifying type and date of an irregularity.

The service center calculates both, a device specific irregularity index and an average index over the same irregularity in the same type of IMD.

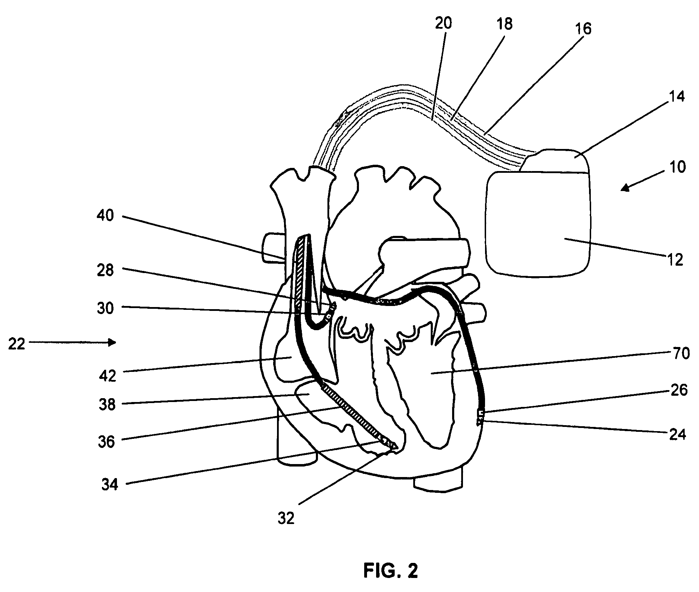

From figure 2 it is apparent that stimulator 10 comprises a case 12 and header 14.

The heart stimulator 10 is connected to three electrode leads, namely a right ventricular electrode lead for 16, a right atrial electrode lead 18 and a left ventricular electrode lead 20. The left ventricular electrode lead 20 is designed to pass trough the coronary sinus of heart 22.

Left ventricular electrode lead 20 comprises a left ventricular tip electrode 24 at the distal end a left ventricular electrode lead 20 and a left ventricular ring electrode 26.

Atrial electrode lead 18 comprises a right atrial tip electrode 28 at the distal end of right atrial electrode lead 18 and a right atrial ring electrode 30.

The right ventricular electrode lead 16 comprises right ventricular tip electrode 32 at the distal end of right ventricular electrode lead 16 and a right ventricular ring electrode 34.

In order to illustrate that heart stimulator 10 may be adapted to act as an implantable cardioverter/defibrillator (ICD) ventricular electrode lead 16 also exhibits a ventricular shock coil 36 for the delivery of defibrillation shocks to right ventricle 38 of heart 22 and an atrial shock coil 40 for the delivery of atrial defibrillation shocks to a right atrium 42 of heart 22.

Each electrode and shock coil of electrode leads 16 to 20 is separately connected to an electric circuit enclosed by case 12 of heart stimulator 10 by way of electrical contacts of a plaque (not shown) at the proximal end of each electrode lead 16 to 20 and corresponding contacts (not shown) in header 14 of heart stimulator 10.

Right atrial shock coil 40 is connected to right atrial shock generator 50 (see figure 2) that is controlled by a control unit 52 of heart stimulator 10.

Similarly right ventricular shock coil 36 is connected to a right ventricular shock generator 54 that is also connected to control unit 52.

Right atrial tip electrode 28 and right atrial ring electrode 30 are both connected to a right atrial stimulation pulse generator 56 and a right atrial sensing stage 58 that internal both connected to control unit 52.

Sensing stages are provided in order to be able to sense a contraction a heart chamber that naturally occurs without artificial stimulation and that is called intrinsic. An intrinsic excitation of a heart chamber results in characteristic electrical potentials that can be picked up via the sensing electrode and that can be evaluated by the sensing stage in order to determine whether an intrinsic excitation - called: intrinsic event - has occurred.

Separate sensing stages 58, 62 and 66 are provided for the right atrium RA 42, right ventricle RV 38 and left ventricle LV 70.

By means of a sensing stage for a heart chamber to be stimulated, the pacemaker is able to only trigger stimulation pulses when needed that is when no intrinsic excitation of the heart chamber occurs in time. Such mode of pacing a heart chamber is called demand mode. In the demand mode the pacemaker schedules an atrial or a ventricular escape interval that causes triggering of an atrial or ventricular stimulation pulse when the escape interval times out. Otherwise, if an intrinsic atrial or ventricular event is detected prior to time out of the respective atrial or ventricular escape interval, triggering of the atrial or ventricular stimulation pulse is inhibited. Such intrinsic (natural, non-stimulated) excitation are manifested by the occurrence of recognizable electrical signals that accompany the depolarization or excitation of a cardiac muscle tissue (myocardium). The depolarization of the myocardium is usually immediately followed by a cardiac contraction. For the purpose of the present application, depolarization and contraction may be considered as simultaneous events and the terms "depolarization" and "contraction" are used herein as synonyms.

Right atrial stimulation pulse generator 56 is adapted to generate atrial stimulation pulses of sufficient strength to cause an excitation of atrial myocardium by an electrical stimulation pulse delivered via right atrial tip electrode 28 and right atrial ring electrode 30. Preferably, means are provided to adapt the right atrial stimulation pulse strength to the stimulation threshold.

Right atrial sensing stage 58 is adapted to pick up myocard cardial potentials indicating an intrinsic atrial excitation that corresponds to a natural atrial contraction. By way of right atrial sensing stage 58, it is possible to stimulate the right atrium 42 of heart 22 in a demand mode wherein a right atrial stimulation pulse is inhibited if an intrinsic atrial event (intrinsic atrial excitation) is sensed by right atrial sensing stage 58 prior to expiration of an atrial escape interval.

In a similar manner, right ventricular ring electrode 34 and right ventricular tip electrode 32 are connected to right ventricular stimulation pulse generator 60 and to a right ventricular sensing stage 62 that in turn are connected to control unit 52. By way of right ventricular tip electrode 32, right ventricular ring electrode 34, right ventricular stimulation generator 60 and right ventricular sensing stage 62, right ventricular stimulation pulses can be delivered in a demand mode to the right ventricle 38 of heart 22.

In the same way left ventricular tip electrode 32 and left ventricular ring electrode 26 are connected to the left ventricular stimulation pulse generator 64 and the left ventricular sensing stage 66 that internal connected to control unit 52 and that allow for stimulating a left ventricle 70 of heart 22.

Triggering and inhibition of delivery of stimulation pulses to the right atrium, the right ventricle or the left ventricle is controlled by control unit 52 in a manner generally known to the man skilled in the art. The timing that schedules delivery of stimulation pulses if needed is controlled by a number of intervals, that at least partly may depend on a hemodynamic demand of a patient that is sensed by means of an activity sensor 72 that is connected to control unit 52. Activity sensor 72 allows for rate adaptive pacing wherein a pacing rate (the rate of consecutive ventricular stimulation pulses for a duration of consecutive atrial stimulation pulses) depends on a physiological demand of a patient that is sensed by a way of activity sensor 72. Details of rate adaptation are known to the man skilled in the art but need not to be explained in detail in this description.

Whereas an actual stimulation rate determines the timing from one (paced) heart cycle to another, intervals like an atrioventricular delay interval and an interventricular delay interval determine the timing within one heart cycle. Starting with an atrial event, the right ventricle would be excited (either intrinsically or paced) at the end of an atrioventricular delay interval. A left ventricular contraction should follow the right ventricular contraction at the end of an interventricular delay interval. This shall include the case, wherein the right ventricle and the left ventricle are excited the same time resulting in an interventricular delay interval duration of zero. Also, it is possible that the left ventricle is excited prior to the right ventricle resulting in an negative interventricular delay interval duration.

In any case, the atrioventricular delay interval duration and the interventricular delay interval duration need to be adapted to an individual heart in order to achieve an optimized cardiac output.

Adaptation of these and other parameters is performed during implantation and during follow-up sessions taking into account irregularities that may have occurred since implantation or a previous follow-up session.

To assist a physician during a follow-up session, either the external device 2 or the remote service center is adapted to allow the following scenario:
1. The physician selects the patient and indicates that he is about to perform a follow-up.
2. The external device 2 or the service center 4 generates a list of irregularities which took place after the previous follow-up so that the physician knows which problems to address. This list of irregularities contains every type of irregularity which was detected by Home Monitoring.
3. For each detected irregularity the external device 2 or the service center 4 calculates an irregularity index which makes it inter-patient comparable or makes comparable for alternative modes or setups of an individual IMD.
4. For each detected irregularity the external device 2 or the service center 4 calculates an average index over the same irregularity. The average index may be calculated over all patients in the external device 2 or the service center 4 which have the same type of implant (given that there is a substantial base of other patients with this implant type) in order to make the index inter-patient comparable.
5. The external device 2 or the service center 4 compares said calculated irregularity index with said calculated average index and displays it to the physician conducting the follow-up if it significantly differs.
6. In that case, the external device 2 or the service center 4 furthermore calculates a list of hints which can help the physician to improve therapy of this irregularity. These hints may comprise
   - static suggestions which are always associated with the irregularity
   - dynamic suggestions: A dynamic suggestion calculates an index of the device setting currently configured to treat the irregularity and either compares it to an average index over all device settings of implants (IMDs) of this type or compares it to a corresponding index for an alternative setup or mode of operation of the same implant.

The external device 2 or the service center 4 gives the physician conducting the follow-up the possibility
- to rate the given hint as unimportant (either for this time or generally)
- to tell the assistant that the issue is being handled

Further, the external device 2 or the service center 4 may give the physician conducting the follow-up the possibility to automatically re-program the implant as suggested.

The external device 2 or the service center 4 groups and priorizes the irregularities according to an internally stored irregularity priority list and presents the irregularities highest priority first.

If the external device is not designed as a stand-alone device but designed to cooperate with the remote service center, follow-up support unit is accessible over any internet-connected computer and for every physician who has the right to view and change the patient's data.

This scenario provides for the following advantages of the invention over the prior art:
1. Patient compliance is no longer necessary. The invention doesn't rely on the patient memorizing or writing down all symptoms.
2. It is no longer necessary for the attending physician to browse through cardio reports collected since the last follow-up examination.
3. The problem of keeping several copies of one patient file up to date and consistent with one another is solved.

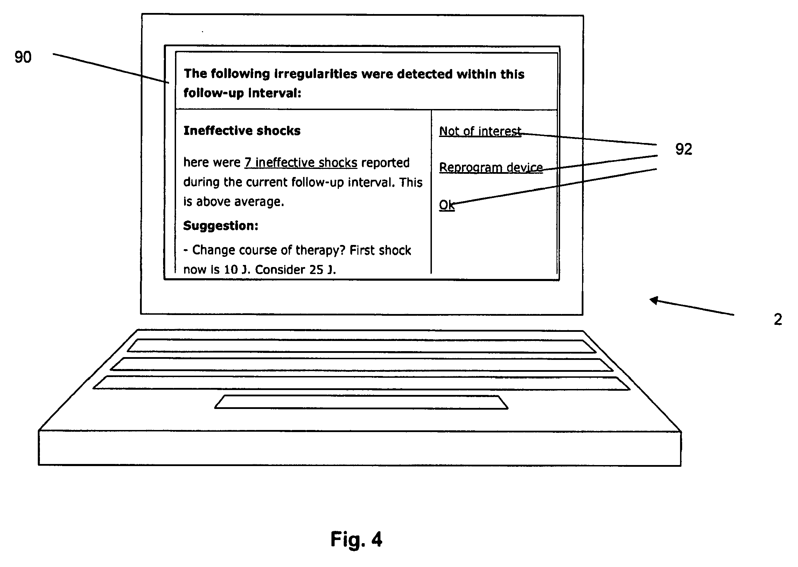

Figure 4 shows a workstation 2 that is used as the external device of figure 1. Workstation 2 comprises a touch sensitive display 90 that allows displaying of the hints generated and of response buttons 92 that can be actuated by a physician.

The following table gives another example of hints o be displayed and possible response buttons:

| **Home Monitoring Service Center Follow-Up Assistant** | | |
|---|---|---|
| **Follow-Up Assistant** | | |
| Patient: | David Labraccio | |
| SN: | 60209071 | |
| Device type: | Lumax 340 HF-T | |
| Implantation: | Sep 13, 2005 | |
| Today: | Jan, 15, 2007 | |
| Date of last follow-up: | Aug, 12, 2006 | |
| Length of follow-up interval: | | 186 days. |
| Average length of follow-up interval in your user group: | | 172 days. |
| Average length of follow-up interval over all patients with this implant type: | | 322 days. |

| **The following irregularities were detected within this follow-up interval:** | | |
|---|---|---|
| **Ineffective shocks** | | Not of interest |
| There were 7 ineffective shocks reported during the current follow-up interval. This is above average. | | Reprogram device |
| **Suggestion:** | | Ok |
| - Change course of therapy? First shock now is 10 J. Consider 25 J. | | |
| **Ineffective ATP** | | Not of interest |
| Only 5% of all conducted ATP ramps were ineffective. But 78% of all conducted ATP bursts were effective. | | Reprogram device |
| **Suggestion:** | | Ok |
| - Change course of therapy? Exchange all programmed ramps into bursts. | | |
| **Many VT1** | | Not of interest |
| 38 ventricular tachycardia (VT1) detected since last follow-up. This is above average (average for this implant type within the same time: 4). | | Reprogram device |
| **Suggestion:** | | Ok |
| - Examine IEGMs for T-wave oversensing | | |
| - Examine patient medication | | |
| - Change programmed VT1 zone (currently: 120 bpm) to 140 bpm. | | |
| **CHF worsened** | | Not of interest |
| The CHF index for this patient changed from 1.3 to 4.5 (on a scale from 1..10). This is above average (average increase for this implant type within the same time: 0.3). | | Ok |
| **Suggestion:** | | |
| - Examine patient medication. | | |

Although an exemplary embodiment of the present invention has been shown and described, it should be apparent to those of ordinary skill in the art that a number of changes and modifications to the invention may be made without departing from the spirit and scope of the invention. In particular, it is possible to implement the features of the claimed transceiver unit into state of the art implantable medical devices such as implantable pacemakers or implantable cardioverter/defibrillator. This invention can readily be adapted to such devices by following the present teachings. All such changes, modifications and alterations should therefore be recognized as falling within the scope of the present invention.

## Claims

1. An IMD follow-up support unit for supporting a follow-up session for an individual implantable medical device (IMD), said IMD follow-up support unit comprising:
a data input for receiving IMD data, said IMD data including an IMD identification code identifying an individual IMD and an IMD type, and IMD irregularity messages including data identifying type and date of an irregularity,
a database for storing IMD data received via said data input,
a follow-up history dataset comprising at least a date of a latest follow-up session for an individual IMD,
a data processor, which is adapted to:
generate a list of irregularities which took place after a previous follow-up session,
calculate an individual IMD irregularity index for each type of irregularity based on IMD irregularity messages associated to an individual IMD by way of said IMD identification code,
calculate for each type of irregularity an average irregularity index over the same irregularity over all IMD irregularity messages associated to the same type of IMD,
compare said calculated individual IMD irregularity index with said calculated average irregularity index and
generate an indication if said individual IMD irregularity index differs from said average irregularity index,
**characterized in that** said indication comprises calculating a list of hints which can help a user to improve a therapy for this type of irregularity.

2. The IMD follow-up support unit of claim 1, wherein said data processor is further adapted to calculate a list of hints upon generating said indication, wherein said list of hints is calculated on the basis of said irregularity messages stored in said data base and wherein said list of hints comprises at least one of:
- static suggestions which are always associated with the irregularity leading to said indication and
- dynamic suggestions, wherein said data processor is adapted to calculate for each dynamic suggestion an IMD setting index of the device settings currently configured to treat said irregularity and to compare said IMD setting index to an average index over all device settings of IMDs of this type.

3. The IMD follow-up support unit of claim 1 or 2, wherein said IMD follow-up support unit is at least remotely connected to an IMD follow-up workstation comprising
a display for displaying said hints calculated by said data processor
input means for entering of commands and/or data by a user, and
a telemetry link adapted for wireless transmission of data to an IMD,
wherein said IMD follow-up support unit is further adapted to
generate display messages that include
a hint generated and
response buttons that allow a user to respond to said hints,
wherein the follow-up system is further adapted to respond to actuation of a response button and to eventually initiate reprogramming of the IMD according to the hint.

4. The IMD follow-up support unit of claim 3, wherein said response buttons can be actuated by means of a computer mouse.

5. The IMD follow-up support unit of claim 3 or 4, wherein said display is touch sensitive and allows direct actuation of the buttons displayed.

6. The IMD follow-up support unit of one of claims 3 to 5, further comprising means for voice control of the follow-up support unit.

7. The IMD follow-up support unit of one of claims 3 to 6, wherein the response buttons generated include response buttons that allow to
- rate a displayed hint as unimportant,
- acknowledge the displayed hint and to prevent further action by the follow-up support unit,
- initiate automatic re-programming of the IMD according to the displayed hint.

8. The IMD follow-up support unit of claim 7, wherein the response buttons generated include response buttons that allow to rate a displayed hint as generally unimportant, either for this time only or generally or as unimportant for one time only or generally.

9. The IMD follow-up support unit of one of claims 3 to 8, wherein the follow-up support unit is adapted to group and priorize the irregularities according to an internally stored irregularity priority list and to initiate displaying of the irregularities of highest priority first.

10. The IMD follow-up support unit of one of claims 3 to 9, wherein said follow-up support unit is part of said work station.

11. A work station of a follow up support system of claim 10, wherein the workstation is an IMD programmer.

## Patentansprüche

1. IMD-Nachbehandlungsunterstützungseinheit zum Unterstützen eines Nachbehandlungstermins für ein einzelnes implantierbares medizinisches Gerät (IMD), wobei die Nachbehandlungsunterstützungseinheit Folgendes umfasst:
- einen Dateneingang zum Empfangen von IMD-Daten, wobei die IMD-Daten einen IMD-Kenncode, mit dem ein einzelnes IMD und ein IMD-Typ bezeichnet wird, sowie Nachrichten zu IMD-Abweichungen umfassen, die Daten umfassen, mit denen eine Art sowie das Datum einer Abweichung angegeben werden,
- eine Datenbank zum Speichern von IMD-Daten, die über den Dateneingang empfangen werden,
- einen Datensatz zum Nachbehandlungsverlauf, der zumindest ein Datum eines letzten Nachbehandlungstermins für ein einzelnes IMD umfasst,
- einen Datenprozessor, der so ausgelegt ist, dass er:
- eine Liste mit Abweichungen erstellt, die nach einem vorangehenden Nachbehandlungstermin aufgetreten sind,
- einen Abweichungsindex für ein einzelnes IMD für jede Art von Abweichung auf der Grundlage von Nachrichten zu IMD-Abweichungen berechnet, die über den IMD-Kenncode einem einzelnen IMD zugehörig sind,
- für jede Art von Abweichung einen durchschnittlichen Abweichungsindex über dieselbe Abweichung über sämtliche Nachrichten zu IMD-Abweichungen berechnet, die derselben Art von IMD zugehörig sind,
- den berechneten Abweichungsindex für ein einzelnes IMD mit dem berechneten durchschnittlichen Abweichungsindex vergleicht, und
- eine Meldung erzeugt, wenn sich der Abweichungsindex für ein einzelnes IMD von dem durchschnittlichen Abweichungsindex unterscheidet,
**dadurch gekennzeichnet, dass** die Meldung ein Berechnen einer Liste mit Hinweisen umfasst, die einem Benutzer helfen können, eine Behandlung für diese Art von Abweichung zu verbessern.

2. IMD-Nachbehandlungsunterstützungseinheit nach Anspruch 1, wobei der Datenprozessor ferner so ausgelegt ist, dass er eine Liste mit Hinweisen beim Erzeugen der Meldung berechnet, wobei die Liste mit Hinweisen auf der Grundlage der Nachrichten zu Abweichungen berechnet wird und wobei die Liste mit Hinweisen Folgendes umfasst:
- statische Empfehlungen, die stets der Abweichung zugehörig sind, die zu der Meldung führt, und/oder
- dynamische Empfehlungen, wobei der Datenprozessor so ausgelegt ist, dass er für jede dynamische Empfehlung einen IMD-Einstellungsindex der Geräteeinstellungen berechnet, die aktuell dafür eingerichtet sind, die Abweichung zu behandeln, und dass er den IMD-Einstellungsindex mit einem durchschnittlichen Index über sämtliche Geräteeinstellungen von IMD dieser Art vergleicht.

3. IMD-Nachbehandlungsunterstützungseinheit nach Anspruch 1 oder 2, wobei die IMD-Nachbehandlungsunterstützungseinheit zumindest über Fernzugriff an eine IMD-Nachbehandlungs-Workstation angeschlossen ist, die Folgendes umfasst:
- eine Anzeige zum Anzeigen der Hinweise, die der Datenprozessor berechnet hat,
- Eingabemittel zum Eingeben von Befehlen und/oder Daten durch einen Benutzer, und
- eine Telemetrie-Verbindung für eine drahtlose Übertragung von Daten an ein IMD ausgelegt ist,
wobei die IMD-Nachbehandlungsunterstützungseinheit ferner so ausgelegt ist, dass sie Anzeigenachrichten generiert, welche
- einen erzeugten Hinweis und
- Schaltflächen umfassen, die einem Benutzer eine Reaktion auf die Hinweise ermöglichen,
wobei das Nachbehandlungssystem ferner so ausgelegt ist, dass es auf die Betätigung einer Schaltfläche reagiert und dass es schließlich entsprechend dem Hinweis eine Umprogrammierung des IMD auslöst.

4. IMD-Nachbehandlungsunterstützungseinheit nach Anspruch 3, wobei die Schaltflächen mittels einer Computermaus betätigt werden können.

5. IMD-Nachbehandlungsunterstützungseinheit nach Anspruch 3 oder 4, wobei die Anzeige berührungsempfindlich ist und eine unmittelbare Betätigung der angezeigten Flächen ermöglicht.

6. IMD-Nachbehandlungsunterstützungseinheit nach einem der Ansprüche 3 bis 5, die ferner Mittel zur Sprachsteuerung der Nachbehandlungsunterstützungseinheit umfasst.

7. IMD-Nachbehandlungsunterstützungseinheit nach einem der Ansprüche 3 bis 6, wobei die erzeugten Schaltflächen Schaltflächen umfassen, mit denen
- ein angezeigter Hinweis als unwichtig beurteilt werden kann,
- der angezeigte Hinweis bestätigt werden und weitere Maßnahmen der Nachbehandlungsunterstützungseinheit verhindert werden können,
- eine automatische Umprogrammierung des IMD entsprechend dem angezeigten Hinweis ausgelöst werden kann.

8. IMD-Nachbehandlungsunterstützungseinheit nach Anspruch 7, wobei die erzeugten Schaltflächen Schaltflächen umfassen, mit denen ein angezeigter Hinweis, entweder nur für dieses Mal oder allgemein, als allgemein unwichtig oder für nur einmal oder allgemein als unwichtig beurteilt werden kann.

9. IMD-Nachbehandlungsunterstützungseinheit nach einem der Ansprüche 3 bis 8, wobei die Nachbehandlungsunterstützungseinheit so ausgelegt ist, dass sie die Abweichungen nach einer intern gespeicherten Prioritätenliste für Abweichungen zusammenfasst und eine Rangfolge dafür festlegt, und dafür sorgt, dass die Abweichungen mit der höchsten Priorität zuerst angezeigt werden.

10. IMD-Nachbehandlungsunterstützungseinheit nach einem der Ansprüche 3 bis 9, wobei die Nachbehandlungsunterstützungseinheit Teil der Workstation ist.

11. Workstation eines Nachbehandlungsunterstützungssystems nach Anspruch 10, wobei die Workstation ein IMD-Programmiergerät ist.

## Revendications

1. Unité de support de suivi pour dispositif médical implantable IMD pour le soutien d'un suivi de session pour un dispositif médical implantable (IMD) individuel, ladite unité de support de suivi pour IMD comprenant :
une entrée de données pour recevoir des données d'IMD, lesdites données IMD incluant un code d'identification d'IMD identifiant un IMD individuel et un type d'IMD, et des messages d'irrégularité d'IMD incluant des données identifiant le type et la date d'une irrégularité,
- une base de données pour stocker des données d'IMD reçues via ladite entrée des données,
- un ensemble de données de l'historique du suivi comprenant au moins une date de la dernière session de suivi pour l'IMD individuel,
- un processeur de données, qui est adapté pour :
- générer une liste d'irrégularités qui sont survenues après une précédente session de suivi,
- calculer un indice d'irrégularité d'un IMD individuel pour chacun des types d'irrégularité sur la base des messages d'irrégularité d'IMD associés à un IMD par l'intermédiaire dudit code d'identification de l'IMD,
- calculer, pour chacun des types d'irrégularité, un indice moyen d'irrégularité pour la même irrégularité pour tous les messages d'irrégularité d'IMD associés au même type d'IMD,
- comparer ledit indice d'irrégularité calculé pour un IMD individuel avec ledit indice moyen d'irrégularité calculé et
- générer une indication si ledit indice d'irrégularité de l'IMD individuel diffère dudit indice moyen d'irrégularité,
**caractérisé en ce que** ladite indication comprend le calcul d'une liste d'indices qui peuvent aider un utilisateur à améliorer le traitement de ce type d'irrégularité.

2. Unité de support de suivi pour IMD selon la revendication 1, dans laquelle ledit processeur de données est en outre adapté pour calculer une liste d'indices à partir de la création de ladite indication, où ladite liste des indices est calculée sur la base desdits messages d'irrégularité stockés dans ladite base de données et où ladite liste des indices comprend au moins l'une des suggestions:
- suggestions statiques qui sont toujours associées à l'irrégularité conduisant à ladite indication et
- suggestions dynamiques, où ledit processeur de données est adapté pour calculer pour chacune des suggestions dynamiques un indice de réglage de l'IMD pour les paramètres du dispositif configurés à ce moment afin de traiter ladite irrégularité et pour comparer ledit indice de réglage de l'IMD à un indice moyen des paramètres de dispositif de tous les IMD de ce type.

3. Unité de support de suivi pour IMD selon la revendication 1, ou 2, dans laquelle ladite unité de support de suivi pour IMD est au moins connectée à distance à une station de travail de suivi de l'IMD comprenant
- un affichage pour afficher lesdits indices calculés par ledit processeur de données
- des moyens de saisie pour entrer des commandes et/ou des données par un utilisateur, et
- une liaison de télémétrie adaptée pour une transmission sans fil des données à un IMD,
où ladite unité de support de suivi de l'IMD est en outre configurée pour générer des messages d'affichage qui incluent
- un indice généré et
- des boutons de réponse qui permettent à un utilisateur de répondre audits indices, où le système de suivi est en outre adapté pour répondre à l'activation d'un bouton de réponse et pour éventuellement initier la reprogrammation de l'IMD selon l'indice.

4. Unité de support de suivi pour IMD selon la revendication 3, dans laquelle lesdits boutons de réponse peuvent être activés au moyen d'une souris d'ordinateur.

5. Unité de support de suivi pour IMD selon la revendication 3, ou 4, dans laquelle ledit affichage est tactile et permet l'activation directe des boutons affichés.

6. Unité de support de suivi pour IMD selon l'une quelconque des revendications 3 à 5, comprenant en outre des moyens pour contrôler par la voix l'unité de support de suivi.

7. Unité de support de suivi pour IMD selon l'une quelconque des revendications 3 à 6, dans laquelle les boutons de réponse générés incluent des boutons de réponse qui permettent de
- classer un indice affiché comme n'étant pas important,
- reconnaître l'indice affiché et d'empêcher une nouvelle action par l'unité de support de suivi,
- initier la reprogrammation automatique de l'IMD selon l'indice affiché.

8. Unité de support de suivi pour IMD selon la revendication 7, dans laquelle les boutons de réponse générés incluent des boutons de réponse qui permettent de classer un indice affiché comme n'étant dans l'ensemble pas important, soit seulement pour cette fois, soit en général, ou comme n'étant pas important seulement pour une fois ou de manière générale.

9. Unité de support de suivi pour IMD selon l'une quelconque des revendications 3 à 8, dans laquelle l'unité de support de suivi est adaptée pour regrouper et établir des priorités dans les irrégularités selon une liste de priorité des irrégularités stockée en interne et initier en premier l'affichage des irrégularités qui ont la priorité la plus élevée.

10. Unité de support de suivi pour IMD selon l'une quelconque des revendications 3 à 9, dans laquelle ladite unité de support de suivi est une partie de ladite station de travail.

11. Station de travail d'un système de support de suivi selon la revendication 10, dans laquelle la station de travail est une unité de programmation d'un IMD.
